# EUROPEAN PATENT APPLICATION

(11) **EP 1 837 334 A1**
(43) Date of publication of application: **26.09.2007**
(21) Application number: 06700853.2
(22) Date of filing: 12.01.2006
(51) Int. Cl.: C07D 301/14, C07D 301/32, C07D 303/04, C07B 61/00

(54) **METHOD FOR PRODUCING PROPYLENE OXIDE**

(30) Priority: 14.01.2005 JP 2005007343
(71) Applicant: Sumitomo Chemical Company, Limited, Tokyo 104-8260 (JP)
(72) Inventor: TSUJI, Junpei, Chiba 260-0021 (JP); ITOU, Yoshiaki, Chiba 299-0128 (JP); SHINOHARA, Koji, Chiba 299-0125 (JP)
(74) Representative: Henkel, Feiler & Hänzel
(86) International application number: PCT/JP2006/300666
(87) International publication number: WO 2006/075777

(57) **Abstract**

A method for continuously producing propylene oxide, which comprises supplying an organic peroxide and propylene to an epoxidation reactor in which a solid catalyst for epoxidation is packed, thereby subjecting them to an epoxidation reaction, the method having a propylene pre-treatment step described below:
propylene pre-treatment step; a step of treating at least a part of propylene supplied to the epoxidation reactor, which comprises removing an oxygen-containing organic compound contained in propylene.

## Description

### Technical Field

The present invention relates to a method for producing propylene oxide. More particularly, the present invention is a method for continuously producing propylene oxide by supplying an organic peroxide and propylene to an epoxidation reactor in which a solid catalyst for epoxidation is packed thereby to subject to epoxidation.

### Background Art

For example, a method for continuously producing propylene oxide by supplying an organic peroxide and propylene to an epoxidation reactor in which a solid catalyst for epoxidation is packed thereby to subject to epoxidation, is publicly known (e.g. JP02-042072 A). However, there were problems that the solid catalyst was destroyed over the break strength of the catalyst because a pressure drop of a layer of the solid catalyst increased with a continuous operation and that the produced amount had to be reduced by control a supply amount of raw materials for avoiding the destruction of the catalyst.

### Disclosure of the Invention

Under such the situations, an object of the present invention is to provide a method for continuously producing propylene oxide by supplying an organic peroxide and propylene to an epoxidation reactor in which a solid catalyst for epoxidation is packed thereby to subject to epoxidation, the method having excellent effects that situations of which the catalyst is destroyed by the pressure drop of the catalyst layer increased with the production and the produced amount is forced to reduce, can be avoided.

Namely, the present invention relates to a method for continuously producing propylene oxide, which comprises supplying an organic peroxide and propylene to an epoxidation reactor in which a solid catalyst for epoxidation is packed, thereby subjecting them to an epoxidation reaction, the method having a propylene pre-treatment step described below:
Propylene pre-treatment step; a step of treating at least a part of propylene supplied to the epoxidation reactor, which comprises removing an oxygen-containing organic compound contained in propylene.

### Brief Description of the Drawing

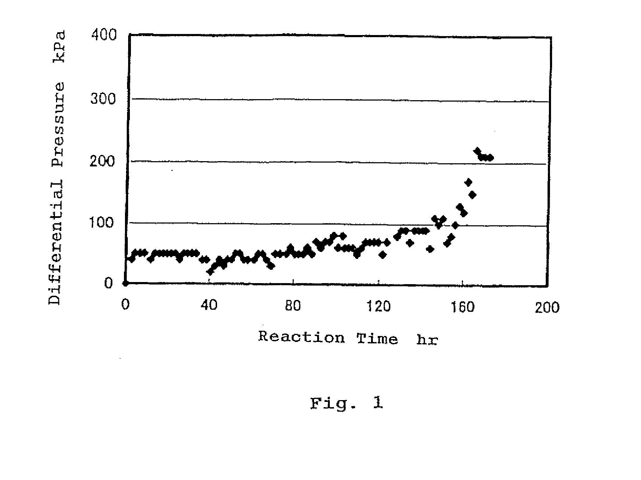

Fig. 1 is a graph showing changes (variation with time) of a pressure difference between the inlet and outlet of a reactor corresponding to a pressure drop of a solid catalyst layer, in Example 1 described after.

### Best Mode for Carrying out the Invention

In the present invention, propylene oxide is continuously produced by supplying an organic peroxide and propylene to an epoxidation reactor in which a solid catalyst for epoxidation is packed, thereby subjecting them to epoxidation reaction.

As a solid catalyst used for the epoxidation reaction, a titanium-containing silicon oxide catalyst is used from the viewpoint of obtaining the desired product under high yield. The catalyst is preferably a catalyst containing titanium chemically bonded to silicon oxide, so-called a titanium-silica catalyst. For example, products carrying a titanium compound on a silica support, products in which a titanium compound is compounded with a silicon oxide by a co-precipitation or sol-gel method or titanium-containing zeolite compounds can be listed.

The particle diameter of the titanium-containing silicon oxide catalyst to be packed in the epoxidation reactor is not particularly restricted, and can be appropriately determined considering the shape and size of the reactor, but is preferably 0.1 to 10 mm, more preferably 0.25 to 5 mm. When the particle diameter is too small, the pressure drop of the catalyst layer may become large, on the other hand, when too large, the yield of propylene oxide may decrease.

As the organic peroxide, cumene hydroperoxide, ethylbenzene hydroperoxide, tert-butyl hydroperoxide and the like are exemplified. The organic peroxide to be supplied to the epoxidation reactor is synthesized by oxidation of a corresponding hydrocarbon compound (for example, cumene, ethylbenzene, isobutane, respectively in organic hydroperoxides described above). Organic acids generated via the oxidation, act as a poison of the epoxidation catalyst.

Therefore, as the organic peroxide to be supplied to the epoxidation reactor, it is preferable to use the peroxide after which the organic acids have been removed. As a removing method, for example , it is preferable to contact with an aqueous solution of a compound containing an alkali metal after or/and during the oxidation reaction. As the compound containing the alkali metal, sodium hydroxide, sodium carbonate, potassium hydroxide, potassium carbonate and the like can be exemplified, and an aqueous solution of any one of these compounds or a mixture thereof can be used. As a concentration of the alkali metal in the aqueous solution, it is preferably 0 . 05 to 10% by weight. When the concentration is too low, removal effect of the organic acids may become insufficient, on the other hand, when too high, the yield may decrease through a promotion of a decomposition reaction of the organic peroxide.

After the contact with the aqueous solution of the compound containing the alkali metal, the resultant is usually separated into an oily phase and an aqueous phase, then the organic acids were removed from the oily phase. However, when the organic peroxide to be supplied to the epoxidation reactor is a one which has contacted with the aqueous solution of the compound containing the alkali metal, a problem that a pressure drop of the solid catalyst layer of the epoxidation reactor increases, may be generated.

Therefore, for further removing the compound containing the alkali metal still remained in the oily phase, it is preferable to separate into an aqueous phase and an oily phase after contacting with water. This operation is repeated if necessary.

A trace amount of water is often contained in the oily phase after separated from the aqueous phase.

Since this water not only lowers yield of the epoxidation reaction, but also acts as a poison of the catalyst, it is preferable to remove water as far as possible. Though, as a method of removing water, a method of removing it using a separation membrane such as a corelesser, a method of removing it by consuming it through a reaction, a method of removing it by distillation and the like are illustrated, removal by distillation is preferable from the industrial viewpoint.

Thus obtained solution containing the organic peroxide is supplied to the epoxidation reactor.

The epoxidation reaction can be carried out in a liquid phase by using a solvent. The solvent is a substance which is liquid at the pressure and temperature under which the reaction is conducted, and must be substantially inert to the reactants and products. The solvent may be a substance existing in the organic peroxide solution to be used. For example, when cumene hydroperoxide is a mixture with cumene as a raw material thereof, ethylbenzene hydroperoxide is a mixture with ethylbenzene as a raw material thereof or tert-butyl hydroperoxide is a mixture with isobutane as a raw material thereof, it can be used as a substitute for the solvent without especially adding a solvent.

The epoxidation temperature is usually from 0 to 200°C, preferably from 25 to 200°C. The pressure may be a pressure enough to keep the reaction mixture liquid. Usually, the pressure is advantageously from 0.1 to 10 MPa.

A molar ratio of propylene to the organic peroxide to be supplied to the epoxidation reactor is preferably 2/1 to 50/1. When the ratio is too low, an efficiency is bad because of lowering of the reaction rate, on the other hand, when too large, a large energy is required in a step of separating and recovering from the epoxidation reaction solution for recycling unreacted propylene of the excess amount.

Though an appropriated linear velocity of a liquid reaction mixture in a fixed bed continuous method is varied depending on composition of the mixture and particle size of the solid catalyst, it is preferably 0.1 to 3 cm/sec in usual. When the linear velocity is below the range, nonuniform flow may occur in the solid catalyst layer of the reactor, on the other hand, when the linear velocity is over the range, the pressure drop of the solid catalyst layer may increase.

Unreacted propylene contained in the liquid reaction mixture after the epoxidation reaction is separated and recovered for recycling to the epoxidation reactor. As a method of separating and recovering unreacted propylene, distillation is used. In the distillation, conditions under which propylene from the liquid reaction mixture is easily vaporized in usual, are used. Though the conditions of the distillation are varied depending on temperature and composition of the reaction mixture to be supplied to a distillation step, a pressure of 0 to 5 MPa, preferably 0 to 3 MPa as a gauge pressure, a overhead temperature of 50 to 150°C and a bottom temperature of 50 to 200°C, preferably 80 to 200°C are illustrated. Further, a method of distillating propylene stepwise using a plurality of distillation column may be adopted.

Thus separated and recovered unreacted propylene can be supplied to the epoxidation reactor after mixing with propylene freshly supplied. However, according to studies of the inventors, there were occurred a problem that, when the propylene supplied to the epoxidation reactor contained unreacted propylene after the epoxidation reaction, the pressure drop of the solid catalyst layer of the epoxidation reactor increased. Further, when an organic peroxide supplied to the epoxidation reactor was contacted with an aqueous solution of a compound containing an alkali metal and propylene supplied to the epoxidation reactor contained unreacted propylene after the epoxidation reaction, there was occurred a problem that the increase of the pressure drop of the solid catalyst layer of the epoxidation reactor was particularly remarkable.

In the present invention, the above-described problem could be solved by adding a pre-treatment step described below to propylene.

The propylene pre-treatment step is a step of treating at least a part of the propylene supplied to the epoxidation reactor, and is a step for removing oxygen-containing organic compounds contained in the propylene. As the oxygen-containing organic compound, formaldehyde, acetaldehyde, formic acid, methanol and the like can be listed.

Much of the oxygen-containing organic compound is generated in the epoxidation reactor. The oxygen-containing organic compounds generated in the epoxidation reactor are contained in unreacted propylene separated and recovered from the liquid reaction mixture after the epoxidation reaction, and are supplied to the epoxidation reactor accompanying the propylene which is recycled to the reactor.

As a method of removing the oxygen-containing organic compounds, there can be listed a method of removing by water washing, a method of removing by distillation, and/or a method of removing by an absorber. Among them, a method of water washing of propylene is preferable because the oxygen-containing organic compounds contained in the propylene can be efficiently removed.

Examples of water washing of the propylene are as follows.

The propylene supplied to the epoxidation reactor is a mixture of unreacted propylene separated and recovered from the liquid reaction mixture after the epoxidation reaction in usual with freshly supplied propylene. As a method of water washing of the propylene, there can be listed a method of contacting gaseous propylene with water, a method of contacting liquid propylene with water, and the like. When that propylene supplied to the epoxidation reactor is usually liquid, contact efficiency, a volume required in contact, and the like are considered, a method of contacting liquid propylene with water is preferred. Liquid propylene is washed by contacting a part or whole of the propylene with water before supplying to the epoxidation reactor. As a method of contacting with water, a method of contacting through mixing in a pipeline by simply injecting water into the pipeline, and a method of raising a contact efficiency with a mixer or the like can be adopted. An example of the mixer includes an agitation mixer composed of an agitator and a container, and a mixer of a pipeline scale such as a static mixer if the contact efficiency is adequate. As contact conditions, any conditions may be used if washing is sufficiently carried out, but as preferable examples, a propylene/waterweight ratio of 0.1 to 200 , a contact temperature of 4 to 120°C, and contact time of 1 to 1800 seconds (excluding a time for still standing and separation) can be exemplified. Propylene mixed and contacted with water is separated into an oily phase and a water phase through still standing.

As preferable conditions for still standing and separation, a still standing and separation time of 1 to 300 minutes and a temperature of 4 to 120°C are exemplified. The concentration of the oxygen-containing organic compounds in propylene after the washing, still standing and separation, as the concentration of each oxygen-containing organic compound, is preferably 200 ppm by weight or less, more preferably 30 ppm by weight or less. The conditions for washing, still standing and separation are selected to obtain such propylene. Thus separated aqueous phase is disposed outside the system because it contains the oxygen-containing organic compounds, or is used another step, if possible. Further, a part thereof may be used again for washing of propylene through recycling. The separated oily phase may be supplied to the epoxidation reactor as it is, but since the residual water not only decreases a yield of the epoxidation reaction but also acts as a poison of the catalyst, it is preferable to remove it as far as possible. As a method of removing water, there can be listed a method of removing by using a separation membrane such as a corelesser, a method of removing by a reaction, a method of removing by using an absorber, a method of removing by distillation, and the like, can be listed. As described above, an increase of the pressure drop of the solid catalyst layer can be suppressed by supplying pre-txeated propylene to the epoxidation reactor.

Further, as described before, propylene oxide from an organic peroxide and propylene can be efficiently produced by the present invention.

### EXAMPLE

The present invention is described by Example below.

### Example 1

A titanium-containing silicon oxide catalyst prepared according to Example 1 of JP 2005-195379 A was screened to obtain the catalyst of 0.25 to 0.5 mm in particle diameter, and then packed in a fixed-bed flow reactor made of stainless steel. A solution containing 25% by weight of cumene hydroperoxide and propylene (formaldehyde concentration of less than 10 ppm by weight) obtained in a pre-treatment step described below were continuously fed under conditions of a weight ratio of 1 : 1 (solution : propylene) and a liquid linear velocity of 2 cm/sec to carry out an epoxidation reaction, and simultaneously, a change of pressure of the inlet and outlet of the reactor was observed. Besides, the reaction temperature was set for 80°C, and the reaction pressure was set for 4.2 MPa in terms of gauge pressure of the outlet of the reactor. In this experiment, the change of a pressure difference with time between the inlet and outlet of the reactor corresponding to a pressure drop was shown in Fig. 1.

In addition, the solution containing 25% by weight of cumene hydroperoxide used above was prepared as follows:
After a reaction liquid obtained by air oxidation was contacted with an aqueous solution containing 2% by weight of an alkali metal compound, the resultant was separated into an oily phase/an aqueous phase. Subsequently, after contacting the oily phase obtained with water, the resultant was separated into an oily phase/an aqueous phase, further the residual water in the oily phase obtained was distilled off to obtain the solution containing 25% by weight of cumene hydroperoxide. Propylene pre-treatment step;
Propylene was water-washed by feeding propylene (containing 50 ppm by weight of formaldehyde) at a rate of 300g/hr, separated and recovered from a mixed solution after the epoxidation reaction and water at a rate of 30 g/hr to a static mixer (contact time: about 5 seconds). In this time, the contact temperature was set for room temperature and the pressure inside the system was set for 2 MPa, and water washing was carried out by contacting liquid propylene with water, subsequently the resultant was separated into propylene and an aqueous phase through standing still. A formaldehyde concentration contained in propylene obtained was less than 10 ppm by weight.

### Industrial Applicability

According to the present invention, there can be provided a method for continuously producing propylene oxide by supplying an organic peroxide and propylene to an epoxidation reactor in which a solid catalyst is packed thereby subjecting to an epoxidation reaction, the method having excellent effects that situations of which the catalyst is destroyed by the pressure drop of the catalyst layer increased with the production and the produced amount is forced to reduce, can be avoided.

## Claims

1. A method for continuously producing propylene oxide, which comprises supplying an organic peroxide and propylene to an epoxidation reactor in which a solid catalyst for epoxidation is packed, thereby subjecting them to an epoxidation reaction, the method having a propylene pre-treatment step described below:
propylene pre-treatment step; a step of treating at least a part of propylene supplied to the epoxidation reactor, which comprises removing an oxygen-containing organic compound contained in propylene.

2. The method according to claim 1, wherein the propylene pre-treatment step is a step of washing propylene with water.

3. The method according to claim 1, wherein propylene supplied to the propylene pre-treatment step is one containing unreacted propylene recovered from a liquid reaction mixture after the epoxidation reaction.

4. The method according to claim 1, wherein the solid catalyst is a titanium-containing silicon oxide catalyst.

5. The method according to claim 1, wherein the organic peroxide supplied to the epoxidation reactor is a one after which an organic acid has been removed.

6. The method according to claim 1, wherein a liquid linear velocity of a liquid reaction mixture in the epoxidation reactor is 0.1 to 3 cm/sec.
